# EUROPEAN PATENT APPLICATION

(11) **EP 1 236 802 A1**
(43) Date of publication of application: **04.09.2002**
(21) Application number: 01103785.0
(22) Date of filing: 16.02.2001
(51) Int. Cl.: C12P 7/02, C12N 1/20, C12N 1/21

(54) **Process for the preparation of perillyl alcohol**

(71) Applicant: AVENTIS ANIMAL NUTRITION S.A., 92160 Antony (FR)
(72) Inventor: Duetz, Wouter Adriaan, 8049 Zurich-Hongg (CH); Witholt, Bernard, 8032 Zurich (CH); Jourdat, Catherine, 69110 Sainte Foy les Lyon (FR)
(74) Representative: Mérigeault, Shona

(57) **Abstract**

The invention provides a process for the preparation of a hydroxymethylated terpene analog, for example (+) or (-) perillyl alcohol, which process comprises contacting a terpene analoog having a terminal methyl group, for example D- or L-limonene, with a microbial cell, which microbial cell is capable of expressing a monooxygenase capable of terminal hydroxylation of an *n*-alkane.

## Description

### Field of the invention

The present invention relates to a process for the preparation of a hydroxymethylated terpene analog from a terpene analog having a terminal methyl group, using a microbial cell or a lysate thereof.

### Background to the invention

Oxidized derivatives of D-limonene, such as carveol, carvone, perillyl alcohol, and perillic acid have a number of applications in, for example, the flavour, flagrance and pharmaceutical industry. In particular, the production of (-) perillyl alcohol is of commercial interest in the light of its proven anticarcinogenic properties when administered at relatively high doses (multiple grams per day). Phase II trials to evaluate perillyl alcohol for the treatment of breast, pancreatic and colorectal cancer are underway.

However, whilst D-limonene is a cheap and widely available chemical, its oxidised derivatives are relatively expensive. One of the reasons for this is that the regiospecific introduction of carbonyl or hydroxy groups into D-limonene by chemical catalysis is notoriously difficult. This is because of the analogous electronic properties of the allylic methylene groups (carbon 1 and 2) and the allylic methyl groups (carbon 5 and 6). Thus, other oxidation products are generated during chemical catalysis, necessitating the further separation of perillyl alcohol from those other oxidation products.

The only microbial enzyme system described so far to be capable of transforming limonene to perillyl alcohol was found in *Bacillus stearothermophilus* BR388. However, this enzyme system is not completely regiospecific (also not if cloned into *E. coli*) and significant quantities of carveol, carvone and terpineol were co-produced (US Patent No. 5,688,673). Since certain physical properties of carveol, perillyl alcohol, and terpineol, such as boiling point and hydrophobicity, are almost identical to those of limonene, the industrial production of perillyl alcohol using this enzyme system is not attractive. Expensive (e.g. chromatographic) purification methods would be needed to obtain sufficiently pure perillyl alcohol.

### Summary of the invention

We have identified a range of *n*-alkane degrading strains of Gram-positive bacteria which are capable of hydroxylating D- or L-limonene highly specifically at the 7-position, leading to the preparation of perillyl alcohol substantially free from other hydroxylation products.

According to the present invention, there is thus provided a process for the preparation of a hydroxymethylated terpene analog, which process comprises contacting a terpene analog having a terminal methyl group with a microbial cell or a lysate thereof, which microbial cell is capable of expressing a monooxygenase capable of terminal hydroxylation of an n-alkane, under conditions such that hydroxylation of the terpene analog having a terminal methyl group to a hydroxymethylated terpene analog occurs.

The invention also provides:
- use of a microbial cell, which microbial cell is capable of expressing a monooxygenase capable of terminal hydroxylation of an n-alkane or a lysate thereof, for use in the preparation of a hydroxymethylated terpene analog;
- a recombinant microbial cell which is capable of expressing a monooxygenase capable of terminal hydroxylation of an n-alkane or a lysate thereof.

### Brief description of the figures

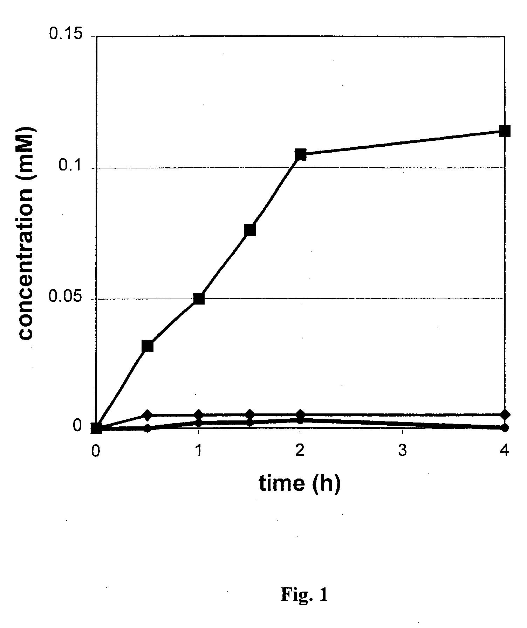

Figure 1 is a graph which shows the kinetics of formation of (+) perillyl alcohol (#), from D-limonene by washed octane-grown cells of *Rhodococcus* sp. ALK2-E1 (3 g dry wt 1⁻¹) . Low concentrations of perillyl aldehyde (!), and perillic acid (⊄) were also formed. No other oxidation products or regioisomers (such as carveol) were detected. The initial specific activity of perillyl alcohol formation was 0.3 U (g dry wt)⁻¹.

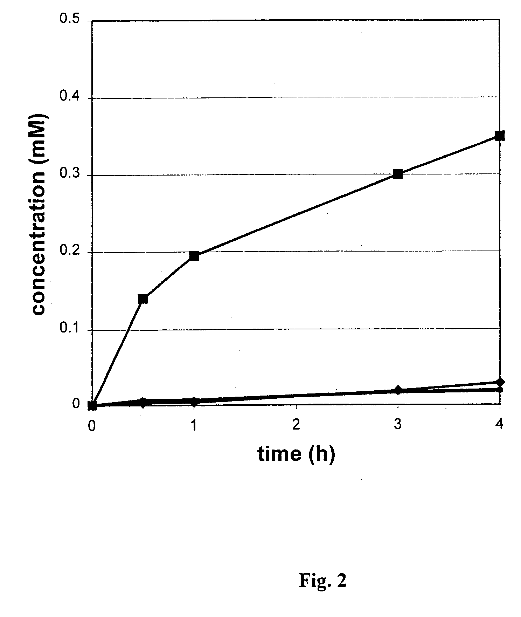

Figure 2 is a graph of the kinetics of formation of (-) perillyl alcohol ( #), from L-limonene by washed octane-grown cells of *Rhodococcus* sp. HXN1900 (0.4 g dry wt 1⁻¹). Low concentrations of perillyl aldehyde (!), and perillic acid (⊄) were also formed. No other oxidation products or regioisomers (such as carveol) were detected. The initial specific activity of perillyl alcohol formation was 6 U (g dry wt)⁻¹.

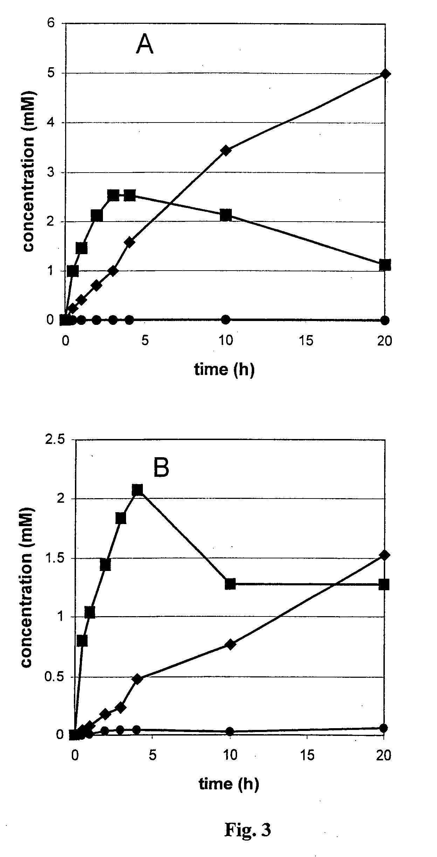

Figure 3 is a graph of the kinetics of formation of perillyl alcohol ( #), perillyl aldehyde (!), and perillic acid (⊄) from D-limonene (A), and L-limonene (B) by washed octane-grown cells of *Mycobacterium* sp. HXN1500 (3 g dry wt 1⁻¹). No other oxidation products or regioisomers (such as carveol) were detected. The initial specific activity of perillyl alcohol formation was 13 and 10 U (g dry wt)⁻¹ for D-limonene and L-limonene respectively.

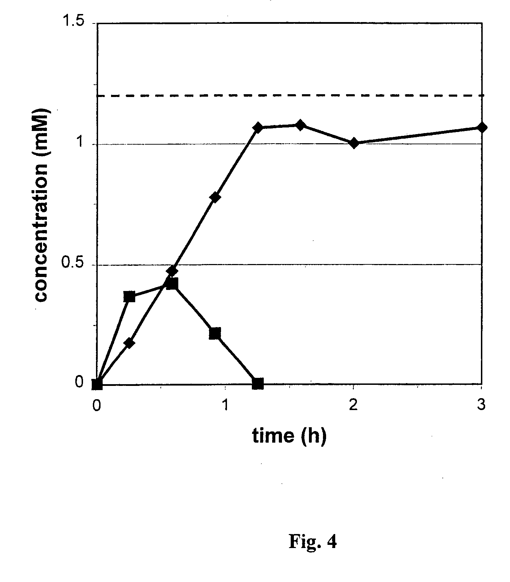

Figure 4 is a graph presenting the yield and kinetics of formation of perillyl alcohol (#), and perillic acid (⊄) from D-limonene by a washed octane-grown cell suspension of *Mycobacterium* sp. HXN1500 (5 ml, 3 g dry wt 1⁻¹) supplied with 1 µl (6.2 µmol) D-limonene. The dashed line indicates the theoretical concentration of the products if the molar yield on D-limonene were 100%. No other oxidation products or regioisomers (such as carveol) were detected.

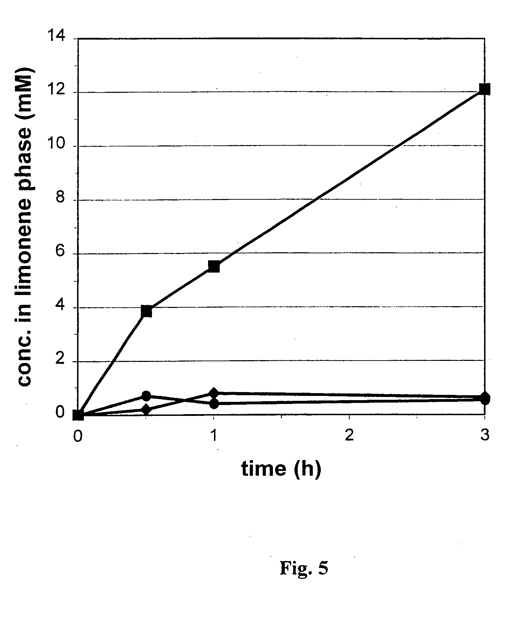

Figure 5 is a graph of the kinetics of formation of (+)perillyl alcohol ( #), and perillic acid (⊄) in the D-limonene phase by a washed octane-grown cell suspension (pH 7.0, 0.4ml) *of Mycobacterium* sp. HXN1500 (0.4 ml, 6 g dry wt 1⁻¹) supplied with 0.1 ml pure D-limonene: practically all perillyl alcohol formed is extracted into the limonene phase. Almost no perillic acid is extracted into the limonene phase since at pH 7.0 more than 99% of the perillic acid is deprotonated and not well soluble in the D-limonene phase. No other oxidation products or regioisomers (such as carveol) were detected.

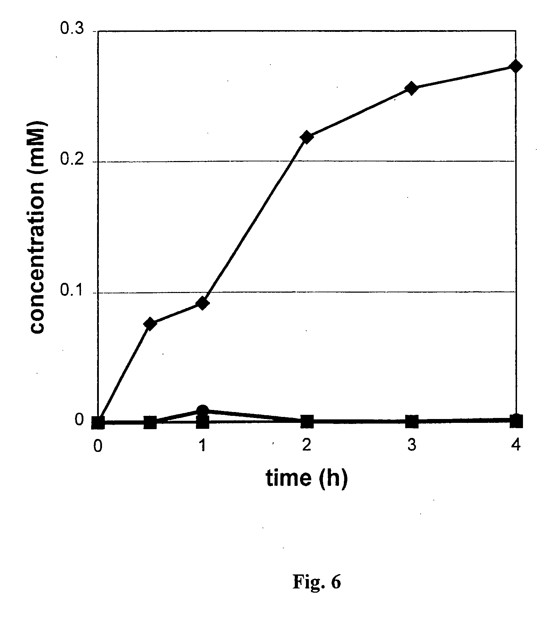

Figure 6 is a graph of the kinetics of formation of perillic acid (⊄) from D-limonene by washed octane-grown cells of *Rhodococcus* sp. ALK2-C7 (3 g dry wt 1⁻¹). Low concentrations of perillyl aldehyde (!), and perillyl alcohol ( #) were also formed. No other oxidation products or regioisomers (such as carveol) were detected. The initial specific activity of perillic acid formation was 0.3 U (g dry wt)⁻¹.

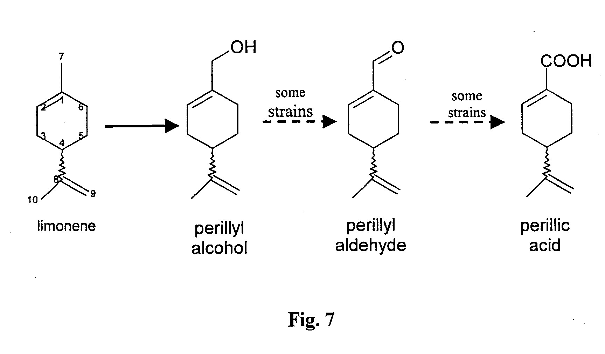

Figure 7 illustrates regiospecific hydroxylation of limonene in the 7-position leading to perillyl alcohol. In some strains the perillyl alcohol formed is through converted to the perillyl aldehyde and perillic acid.

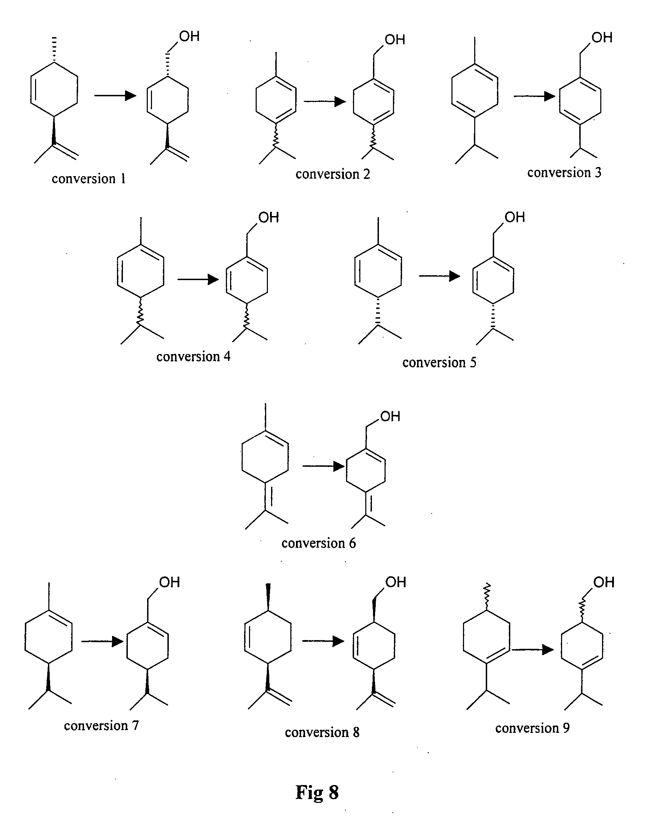

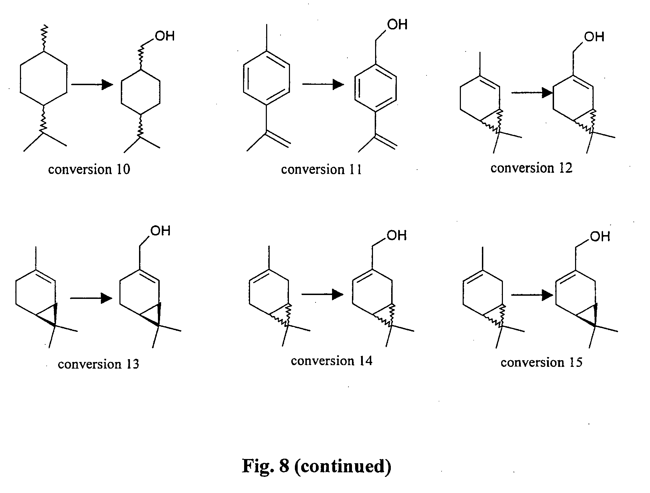

Figure 8 illustrates compounds with an identical or similar carbon skeleton as limonene that were also hydroxylated at the methyl group (C7) with induced cells of *Mycobacterium sp.* HXN-1500. For a list of names of the starting compounds, see the text of Example 4.

### Detailed description of the invention

The process of the present invention permits the production of a hydroxymethylated terpene analog, for example perillyl alcohol, from a terpene analog having a terminal methyl group, for example D- or L- limonene, which is substantially free from other hydroxylation products. The process is believed to be the only process to date which uses microbial enzymes to regiospecifically hydroxylate terpene analogs having a terminal methyl group, for example D- or L- limonene. Significant yields of, for example, perillyl alcohol can be obtained from both D-limonene and L-limonene accompanied by substantially no coproduction regioisomers, such as carveol, piperitenol, isopiperitenol or terpineol. The process is relatively simple, making it attractive for use in industrial-scale application.

The process of the invention comprises contacting a terpene analog having a terminal methyl group, for example, D- or L-limonene, with a microbial cell or a lysate thereof, which microbial cell is capable of expressing a monooxygenase capable of terminal hydroxylation of an n-alkane, under conditions such that hydroxylation of the terpene analog having a terminal methyl group, for example D- or L-limonene, to a hydroxymethylated terpene analog, for example (+) or (-) perillyl alcohol, occurs.

A microbial cell suitable for use in the invention can be identified by screening cultures of candidate microbial cells for an ability to degrade an n-alkane, for example octane. Typically, this can be carried out by determining whether the microbial cell being screened can grow on on an n-alkane supplied as the sole source of carbon. Thus, a cell to be screened may be placed in or on a mineral medium with no added source of carbon other than an n-alkane (or n-alkanes). Generally, it will be convenient to carry out such a screening reaction in a single well of a microtitre plate and therefore many assays may be carried out simultaneously.

Bacteria identified as capable of degrading an n-alkane in the screen set out above may subsequently be screened for an ability to catalyse conversion of, for example D- or L-limonene to (+) or (-) perillyl alcohol. Thus, cell cultures of a candidate microbial cell may be grown in the presence of D- or L- limonene, for example, and also typically in the presence of one or more *n*-alkanes as the sole carbon source, for example *n*-octane. After growth of the microbial cell culture in the present of D- or L-limonene, the cell culture supernatant can be isolated and analysed using any suitable technique. HPLC analysis, for example, will allow the amount, if any, of perillyl alcohol formed during the assay to be determined. Again, it will generally be convenient to carry out such a screening reaction in a single well of a microtitre plate and therefore many assays may be carried out simultaneously.

The microbial cell is capable of expressing a monooxygenase capable of terminal hydroxylation of an *n*-alkane. That is to say, the microbial cell used should be of a strain capable of degrading n-alkanes through a pathway which involves the terminal monooxygenation of *n*-alkanes, generally as an initial degradation step. The *n*-alkane may be a hydrocarbon, having for example 6 to 16 carbon atoms. Generally, the hydrocarbon may be linear hydrocarbon. Preferably, the *n*-alkane is octane. Furthermore, a microbial cell suitable for use in the invention should be capable of hydroxylating D- or L- limonene or an analog thereof specifically in the 7-position.

The microbial cell may be a naturally-occurring cell, typically in an isolated form, or alternatively may be a recombinant cell. The term recombinant cell is taken to include a cell which has been engineered to comprise a polynucleotide which the naturally-occurring form of the cell does not. Typically, that polynucleotide will encode a monooxygenase, which the recombinant cell will be capable of expressing. In addition, the term recombinant cell is taken to include a daughter cell derived from the engineered cell and subsequent generations of cell derived from that daughter cell.

A microbial cell suitable for use in the invention may be eukaryotic, for example a fungal or yeast cell, or may be prokaryotic, for example a bacterial cell. Bacterial cells are preferred. Suitable bacterial cells may be Gram-negative or, preferably, Gram positive. An example of suitable Gram-positive cell is a CNM bacterial cell, in particular a cell from the genus *Corynebacterium, Arthrobacter, Rhodococcus, Nocardia* or *Mycobacterium.* Specific examples of such cells include *Rhodococcus* sp. ALK2-E1, *Rhodococcus* sp. HXN-1900, and *Mycobacterium* sp. HXN1500. *Mycobacterium* sp. HXN1500 was deposited at the Centraalbureau voor Schimmelcultures (CBS) on 13th February 2001 under Accession No. CBS 109282. Alternatively, the Gram-negative bacterial cell can be from the genus *Sphingomonas*, for example a *Sphingomonas* sp. HXN200 cell.

The microbial cell may be one obtained through mutagenesis of a naturally-occurring cell. That is a microbial cell suitable for use in the invention may be a mutant version of a naturally-occurring cell. Mutagenesis of a naturally-occurring cell which is suitable for use in the invention may permit the identification of mutant microbial strains which are even more suitable for use in the invention than their naturally-occurring counterparts. That is to say, the mutant strains may be capable of catalysing higher reaction rates, i.e. convert a greater number of molecules of limonene to perillyl alcohol, may show greater substrate specificity or may show greater specificity for hydroxylation of the 7-position of limonene than the naturally-occurring cells from which they are derived.

A suitable mutant microbial cell for use in the invention may be generated by random mutagenesis, for example by the use of chemical mutagens or alternatively, targeted mutagenesis may be carried out. In targeted mutagenesis, genes encoding monooxygenases may be specifically mutated, for example the active site may be targeted. Mutagenesis can allow microbial cells to be obtained which show improved characteristics in respect of their suitability for use in the present invention when compared to the naturally-occurring cells from which they are derived.

Mutagenesis techniques are well known to those skilled in the art. Generally, the microbial cell on which mutagenesis is carried out is one of the cells described above. The cells obtained from mutagenesis experiments can be screened using the screens set out above.

More than one round of mutagenesis may be carried out, for example two, three, five or ten rounds, and improved strains of microbial cell (in respect of their n-alkane degrading and/or limonene converting activity) may be selected after the completion of each round. Cells identified as having improved properties after a round of mutagenesis will generally be used as starting cells for a subsequent round of mutagenesis. Thus, the invention provides a microbial cell derived via mutagenesis, which is suitable for use in a process of the invention.

A microbial cell for use in the invention, for example a bacterial cell, may be a recombinant cell. A recombinant cell of the invention will comprise a monooxygenase-encoding polynucleotide, Athe introduced polynucleotide@, in addition to any monooxygenase-encoding polynucleotide which the naturally-occurring form of the recombinant cell may comprise. The introduced polynucleotide may have the same sequence as a monooxygenase-encoding polypeptide which the naturally-occurring form of the recombinant cell comprises. Alternatively, the introduced polynucleotide may have a different sequence from any monooxygenase-encoding polypeptide polypeptide which the naturally-occurring form of the recombinant cell comprises.

The introduced polynucleotide may be a naturally-occurring monooxygenase-encoding polynucleotide, for example a monooxygenase-encoding polynucleotide isolated from one of the bacteria described above. Alternatively, the introduced polynucleotide can be a variant sequence of a naturally-occurring monooxygenase-encoding polynucleotide. For example, a naturally-occurring monooxygenase-encoding polynucleotide may be modified by nucleotide substitutions, for example from 1, 2 or 3 to 10, 25, 50 or 100 substitutions to give a polynucleotide suitable for use as an introduced polynucleotide. The introduced polynucleotide may alternatively or additionally be modified by one or more, for example from 2, 5 or 10 to 20, 25, 50 or 100 insertions and/or deletions and/or an extension at either or both ends as compared to a a naturally-occurring monooxygenase-encoding polynucleotide. Degenerate substitutions and/or substitutions which would result in a conservative amino acid substitution when the modified sequence is translated may be made to a naturally-occurring monooxygenase-encoding polynucleotide to obtain a polynucleotide suitable for use as an introduced polynucleotide. The introduced polynucleotide will of course always encode a polypeptide which has monooxygenase activity.

Naturally-occurring monooxygenase-encoding polynucleotides may be isolated from any suitable source, in particular from one of the bacteria set out above. The monooxygenase-encoding polynucleotide should be obtained from a microbial cell capable of accepting one or more *n*-alkanes as a source of carbon and energy. Those skilled in the art will be able to readily obtain such polynucleotides. Modified versions of such naturally-occurring polynucleotides can be readily obtained by those skilled in the art according to well-known techniques.

The introduced polynucleotide should be capable of being expressed in a recombinant cell. Thus, the introduced polynucleotide will typically be operably linked to a control sequence which is capable of providing for the expression of the introduced sequence in the recombinant cell. The term Aoperably linked@ refers to a juxtaposition wherein the components described are in a relationship permitting them to function in their intended manner. A regulatory sequence, such as a promoter Aoperably@ linked to a coding sequence is positioned in such a way that expression of the coding sequence is achieved under conditions compatible with the regulatory sequence.

Promoters and other expression regulation signals may be selected to be compatible with the microbial host cell for which expression is designed. It may be convenient to use an actual monooxygenase gene promoter or alternatively, it may be more convenient to use a promoter which permits high level and/or consitutive expression of the introduced polynucleotide. Examples of the latter type of promoter include the bacterial *lac*Z, T7 and T3 promoters, the *S. cerevisiae* GAL4 and ADH promoters and the *S. pombe nmtl* and *adh* promoters.

The introduced polynucleotide may be introduced into a host microbial cell in any convenient manner. Typically, the introduced polynucleotide and a promoter linked operably thereto are incorporated into a vector, which is then introduced, by for example, transformation or transfection, into the host microbial cell of choice. Any suitable vector may be used, for example a plasmid, virus or phage vector. The vector may provide an origin of replication, one or more selectable marker genes, for example an ampicillin resistance gene in the case of a bacterial plasmid. The vector may provide for transient expression in the recombinant microbial cell or may provide for stable expression, i.e. the introduced polynucleotide becomes incorporated into the chromosomal DNA of the recombinant cell.

Thus, the invention provides a recombinant microbial cell suitable for use in a process of the invention. Such a recombinant microbial cell may be mutagenised as has been described above.

A terpene analog having a terminal methyl group suitable for use in the invention is, for example, a cyclic monoterpene having a carbon skeleton similar to, or preferably identical to, that of limonene. The structure of limonene is set out in Figure 7. According to a feature of the invention there is provided a process for the seletive terminal hydroxylation of a terpene analog having a terminal methyl group, generally having 10 carbon atoms, typically with 0 to 4 double bonds, especially 1, 2 or 3 ring double bonds and typically one or two rings to convert a terminal methyl group to a terminal hydroxymethyl group. It is possible for one of the rings to be formed as a bridge containing, typically 1 carbon atom. In other words, it can be formed by a direct link between 2 carbon atoms of an existing ring or with an intermediate methylene group. It is also possible for a cyclopropane ring to be fused with a six-membered ring.

A terpene analog having a terminal methyl group suitable for use in the invention will typically have a total of 2 or 4 exocyclic methyl or methylene groups, for example 2 methyl groups and 1 methylene group.

Oxidation in a process of the invention causes the formation of a C-O bond in the compound, typically as the hydroxide from the oxidation of a carbon-hydrogen bond. In certain circumstances it is preferred that further oxidation of the hydroxy group occurs, so that the hydroxy group is converted into an aldehyde or ketone group. Typically, only the carbon at position 7 of the terpene analog having a terminal methyl group is attacked in the process of the invention. In addition, the process typically preserves the chiral centre. Thus, when the substrate in the invention consists of a single enantiomer, the product typically consists of a single corresponding enantiomer, or contains a preponderance of the corresponding enantiomer.

Particular examples of a terpene analog having a terminal methyl group suitable for use in the invention include pinene, terpinene, sabinene and thujene. Further specific examples of analogs which are suitable for use in the invention are (1R)-(+)-trans-isolimonene (CAS 5113-87-1), alpha-terpinene (CAS 99-86-5), gamma-terpinene (CAS 99-85-4), alpha-phellandrene, racemic mixture (CAS 99-83-2), R(-) alpha- phellandrene (CAS 4221-98-1), terpinolene (CAS 586-62-9), (+)-*p*-meth-1-ene (CAS 1195-31-9), (+)- trans-*p*-menth-2-ene (CAS 5113-93-9), 4-methyl-1-iso-propylcyclohexene (CAS 500-00-5), *p*-menthane (CAS 99-82-1), dehydro p-cymene (CAS 1195-32-0), 2-carene (CAS 554-61-0), (+)-2-carene (CAS 4497-92-1), delta-3-carene (CAS 13466-78-9) or (+)-3-Carene (498-15-7).

In the process of the invention, a terpene analog having a terminal methyl group is typically contacted with a cell culture of a microbial cell capable of expressing a monooxygenase capable of terminal hydroxylation of an *n*-alkane of a lysate thereof. Typically, the cell culture or cell lysate is a culture of a single type of microbial cell or lysate thereof. However, it is possible to carry out a process of the invention using a cell culture or lysate that comprises more than one type of microbial cell or cell lysate. The growth medium used for cell culture may be any suitable mineral medium and will include an assimilable carbon and nitrogen source. Commercially available culture media for cell culture are widely available and can be used in accordance with the manufacturers instructions The presence of an inducer may be required, for example an *n*-alkane, for example octane. Clearly, if a recombinant cell is used, which expresses a monooxygenase-encoding polynucleotide under the control of a constitutive promoter, an inducer will not need to be included in the growth medium. The process is generally carried out under aerobic conditions.

Typically, the terpene analog having a terminal methyl group, for example D- or L-limonene, is contacted with the microbial cell under conditions suitable for hydroxylation to a hydroymethylated terpene analog, for example (+) or (-) perillyl alcohol, to occur. Thus, the a terpene analog having a terminal methyl group is typically contacted with the microbial cell at a temperature of from 15°C to 40°C, preferably from 20°C to 37°C.

The process of the invention may also be carried out by contacting a terpene analog having a terminal methyl group with a lysate of a microbial cell described herein. That is, the terpene analog having a terminal methyl group may be contacted with a cell-free extract in a process of the invention. Methods for producing cell lysates are well known to those skilled in the art and any suitable method may be used.

The D- or L- limonene, for example, is preferably provided to a microbial cell culture medium or lysate in the gas phase or as a second liquid phase, which is either pure or dissolved in an inert solvent, for example hexadecane. Typically, the limonene is added directly to the culture medium in small increments over time. Techniques for carrying out such a process are well known in the art.

Typically, in a process of the invention hydroxylation occurs so that a hydroxymethylated terpene analog, for example perillyl alcohol, is the major oxidation product. Thus, the hydroxymethylated terpene analog will generally constitute at least 60%, at least 70%, at least 80%, at least 90%, at least 95% or at least 99% of the total amount of oxidation products, where the percentage of the total amount of oxidation products is expressed in terms of the percentage of the summed molar concentrations of the oxidation products. Preferably, there will be substantially no production of oxidation products other than the hydroxymethylated terpene analog, for example perillyl alcohol.

The hydroxymethylated terpene analog product may be isolated from the culture medium by solid phase extraction or by extraction with a suitable organic solvent, for example butanol, chloroform or any other suitable hydrocarbon. If limonene, for example, is used in the pure form, the product may be continuously extracted in the limonene phase. The limonene that is not converted may be removed by distillation under reduced pressure.

If the terpene analog having a terminal methyl group, for example limonene, is added as a significant second phase (eg. 10-25% of the aqueous phase) there will be relatively little of the mydroxymethylated terpene analog, for example perillyl alcohol, through converted to the aldehyde and the acid, simply because the aqueous concentration of the alcohol is relatively low). If limonene, for example, is added in small (limiting) amounts this has two effects both leading to more acid formation: firstly, the specific perillyl alcohol formation will be lower (mass transfer limitation of limonene to the cells); and secondly, the perillyl alcohol concentration in the aqueous phase will be higher (no extraction in the limonene phase because there is none) thus saturating the dehydrogenase responsible for the further oxidation to the aldehyde and the acid to a higher extent. More through conversion to the acid may be desired for the production of perillic acid, for example.

### The following Examples illustrate the invention:

### Examples

### Materials and Methods

**Bacterial strains.** 137 alkane degrading strains were obtained from different sources. For the source and reference of the following 18 strains, see Table 1 of Smits *et al*. (1999, Environ. Microbiol. 1, 307-318): *P. fluorescens* CHA0, *Burkholderia cepacia* ATCC 25415, *P. putida (oleovorans)* GPol (ATCC 29347), *P. putida* P1, *Pseudomonas aeruginosa* KSLA 473, *P. aeruginosa* ATCC 17423, *P. aeruginosa* 196Aa, *P. aeruginosa* Sol 20, *P. aeruginosa* PAO1, P. *aeruginosa* PAO1S, *Rhodococcus rhodochrous* NCIMB 12566, *R. erythropolis* NRRL B-16531, *Prauserella (Amycolatopsis) rugosa* NRRL B-2295, *Acinetobacter* sp. ADP1, *Acinetobacter* sp. 2769A, *Acinetobacter calcoaceticus* 69-V, *A. calcoaceticus* EB104, *A. calcoaceticus* NCIB 8250.

Strains 1-D, 19-D, 23-D, 30-D, 7-H, 97-H, 114-H, 115-H, 1-O, 35-O, 36-O, 42-O, 43-O, 62-O, 65-O, 66-O, 4-S, 139-S, 154-S, 18-V, 33-V, 50-V, and 51-V were a gift from Susanne Schorcht (Schorcht, 1998, Mikrobiologische und molekularbiologische Charakterisierung alkaanabbauender Bakteriengemeinschaften. In Biologie/Chemie. Bremen: Universität Bremen). The strains were isolated from soil samples, obtained from 10 cm depth near a garage in Bremen, Germany. The samples were resuspended, shaken with glass beads in sterile Erlenmeyer flasks, filtered and plated in a dilution series on rich medium or a minimal medium with octane (O), hexadecane (H), diesel (D), squalene (S) or nutrient broth (V) as C-sources. Fifty-five strains were isolated, named according to the growth substrate, and partially characterized (Schorcht, 1998, *supra).* Of these strains, twenty-three were included in this study. Six Gram-positive strains which appeared relatively diverse and stable, and grew well on *n*-alkanes, were further characterised (35-O, 62-O, 23-D, 50-V, 115-H and 154-S). Partial 16S rDNA sequences were obtained with amplification primers 16F27 and 16R1525, and sequencing primers 16F355 and 16R519 (Hauben *et al*., 1997, Int. J. Syst. Bacteriol. 47, 328-335; Karlson *et al*., 1993, J. Bacteriol. 175, 1467-1474). The 16S sequences of 115-H and 154-S were identical to each other, and to those of several *R. fascians* strains, while the 16S sequences of 23-D, 35-O, 50-V and 62-0 were identical to the 16S sequence of the R. *erythropolis* type strain ATCC 4277T (X81929).

Strains R1, R4, PN1, PN2, PN3, PR1, PR2, and PR3 were isolated from soil, water, and meadow samples taken at various sites in The Netherlands, but not identified (J. B. van Beilen, unpublished results).

Strains HXN 100 (CNM-group), HXN 200 (*Sphingomonas* sp.), HXN 300, HXN 400 (*P. aeruginosa*), HXN 500 (CNM-group), HXN 600 (CNM-group), HXN 1000 (CNM-group), HXN 1100 *(P. mendocina* lineage), HXN 1200 (CNM-group), HXN 1300, HXN 1400, HXN 1500 (*Mycobacterium* sp.), HXN 1600, HXN 1700, HXN 1800, HXN 1900 (CNM-group), and HXN 2000 (CNM-group) were isolated from a hexane air-filter in Stuttgart, and partially identified based on Gram-staining, morphology, metabolic capabilities and fatty acid analysis (Plaggemeier, 2000, Elimination der schwer wasserlöslichen Modellabluftinhaltssoffe n-Hexan und Toluol in Biorieselbettverfahren. In Institute für Siedlingswasserbau, Wassergüte, und Abfallwirtschaft. Stuttgart: Universität Stuttgart: Universität Stuttgart; and T. Plaggemeier and K.-H. Engesser, unpublished results).

Strains BC20 (Gram-negative), BCG50 (Gram-positive), and BCG51 Gram-positive) were a gift of Bioclear, Groningen, The Netherlands. *Pseudomonas citronellolis* DSM 50332 and *P. fluorescens* ATCC 15453 were obtained from the DSM and ATCC strain collections.

An additional 73 alkane degrading strains were isolated from soil, water, and sewage sludge samples and leaves from trees various sites in Switzerland for their ability to grow with octane as the sole carbon and energy source, and were subjected to basic taxonomic tests like microscopic studies and Gram-stains, and the capability to grow with glucose, hexane, octane, decane, dodecane and hexadecane as sole sources of carbon and energy supplied via the gas phase. Alk2-A2 and Alk2-A3, Alk2-B8, Alk2-B9, Alk2-B10, Alk2-B11, Alk2-B12, Alk2-C1, Alk2-C2, Alk2-C4, Alk2-C5, Alk2-C6, Alk2-C7, and Alk2-G2 were isolated from soil samples. Alk2-A4, Alk2-A5, Alk2-A6, Alk2-A7, Alk2-A8, Alk2-A9, Alk2-A10, Alk2-A11, Alk2-A12, Alk2-B1, Alk2-B2, Alk2-B3, Alk2-B4, Alk2-B5, Alk2-B6, Alk2-B7, and Alk2-G3 were isolated from a sewage treatment plant in Kaisten Rucklauf (Switzerland). Alk2-C8, Alk2-C9, Alk2-C10, and Alk2-C11 were isolated from the leaves from a Hawthorn tree (Crataegus monogyna). Alk2-D1, Alk2-D2, Alk2-D3, Alk2-D4, Alk2-D5, Alk2-D6, Alk2-D7, Alk2-D8, Alk2-D9, Alk2-D10, Alk2-D11, Alk2-E1, Alk2-G4, and Alk2-G5 were isolated from the leaves from an Ash tree (Fraxinus excelsior). Alk2-E2, Alk2-E3, Alk2-E4, Alk2-E6, Alk2-E7, Alk2-E8, Alk2-E9, Alk2-E10, and Alk2-E11 were isolated from the leaves of an Oak tree (genus Quercus). Alk2-E12, Alk2-F1, Alk2-F2, Alk2-F3, Alk2-F4, Alk2-F5, Alk2-F6, Alk2-F8, Alk2-F9, Alk2-F10, Alk2-F11, Alk2-G6, and Alk2-G7 were isolated from leaves of a Lime tree (Tilia vulgaris). Alk2-F12, and Alk2-G1 were isolated from needles from a pine tree.

**Screening.** Multiple strains stored in one individual 96 well microtiter plate at B80 °C were sampled simultaneously (without thawing the remaining culture) using a spring-loaded 96-pin replicator (Kuhner, Basel, Switzerland) as previously described (Duetz *et al*., 2000, Appl. Environ. Microbiol. 66, 2641-2646) and transferred to a regular sterile polystyrene microtiter plate (type 3072, Costar, Cambridge, Mass.) of which each well (working volume 350 µl) was filled with 180 µl of a solidified agar (2% [w/v]) mineral medium without any added C-source. The inoculated microtiter plate (lid on top was kept at a distance of 2 mm from the wells, in order to allow for a sufficient level of gas diffusion in and out the wells) was placed in a dessicator together with a beaker of water and a 50 ml beaker containing 10 ml of 1:1:1:1:1 mixture (v/v) of hexane, octane, decane, dodecane, and hexadecane.

After 7 days of growth at ambient temperature (28 °C), the cell mass which had developed on the agar surface was harvested as follows. 110 µl of a K₂HPO₄/KH₂PO₄-buffer (50 mM, pH 7.0) was added to each well. Repeated lateral movement of the spring-loaded replicator in the wells resulted in the suspension of a large part of the cell mass. The suspensions were subsequently transferred (using a 12 channel multipipette and wide orifice tips) to a microtiter plate with 0.5 ml conical wells (Maxi-plaque, Polylabo, Geneva). The microtiter plate was centrifuged for 15 min at 4000 rpm in a centrifuge (Eppendorf, type 5403). After disposal of the supernatant, the cells were resuspended in 100 µl of a K₂HPO₄/KH₂PO₄-buffer (50 mM, pH 7.0) containing 100 mM glucose by repeated filling and emptying of wide-orifice pipette tips (using a 12 channel multipipette). Subsequently, 2 µl of optically pure D-limonene or L-limonene (Fluka, p.a.) was added to each well and the wells were closed using a sandwich cover (a pierced layer of soft silicone in combination with a rigid polypropylene plate) as described previously (Duetz *et al*., 2000, *supra),* followed by 2 hours of orbital shaking at 300 rpm, 5 cm shaking amplitude, at 25 °C. The microtiter plate was then centrifuged for 15 min at 4000 rpm. 50 µl of the supernatant (without the remaining D-limonene phase) was transferred into a half-area microtiter plate (Costar type 3696, Corning, NY) using a 12 channel multipipette.

**Analysis by HPLC-MS.** Analysis was performed using a HPLC (Agilent 1100 series) equipped with a diode array detector and a mass detector in series. Solvent 1 was acetonitrile/methanol (1:1 [vol/vol]) + 0.1 % formic acid. Solvent 2 was water + 0.1 % formic acid. Solvents 1 and 2 were used in a 60:40 [vol/vol] ratio for 10 minutes followed by a steep gradient to 90% solvent 1, at a total run time of 13.2 minutes, using a Machery Nagel C18 column (50 x 3 mm) equipped with a pre-column of the same material with a length of 8 mm. The settings of the MS were as follows: APCI mode, positive ionization, fragmentor voltage 50 V, gas temperature 350<C, vaporizer temperature 375<C, drying gas (N2): 4 1 min-1, nebulizer pressure 50 psi, capillary voltage 2000 V, corona current 6 µA. The MS was run in the selected ion mode (SIM) set to detected the following masses (relative dwell times in parentheses): 135 (40%), 137, (5%), 151, (10%), 153 (7%), 176 (15%). Perillic acid did not give a good MS signal at any mass but could be sensitively quantified by the DAD signal at 225 nm. The characteristic UV spectrum was helpful in confirming the presence of perillic acid.

**Growth of** *Mycobacterium sp.* **HXN1500 in a fed-batch bioreactor.** *Mycobacterium* sp. HXN1500 was grown on a mineral medium (Duetz *et al*., 2000, *supra)* supplied with 5 mM glucose in a 3-liter bioreactor. At inoculation, the working volume was 1200 ml and the optical density at 540 nm (OD540) was 0.1. The reactor was stirred at 600 rpm. Octane-saturated air was supplied to the reactor at 11 min⁻¹. The reactor was continuously fed with fresh mineral medium without any C-source at a rate of 12 ml h⁻¹. The OD540 gradually increased to a level of 2.4. After 5 days, 11 cell suspension was harvested, centrifuged, and the pellet was stored at B30 <C for later use in biotransformation assays. Two days later, the rest of the culture (1.5 1) was harvested and cell pellets were stored in quantities of 80 mg dry wt at B30 <C.

**Biotransformation kinetics of D-limonene and L-limonene by chemostat-grown *Mycobacterium sp.* HXN1500.** Frozen cell pellets of reactor-grown *Mycobacterium sp.* HXN1500 were resuspended in a K₂HPO₄/KH₂PO₄-buffer (50 mM, pH 7.0) to an OD₅₄₀ of 10-12. Quantities of 0.5 ml of this cell suspension were transferred to a number of 2.4 ml wells of a square deepwell microtiter plate (Riplate, Ritter, Germany) that was detoxified and flattened as described previously. Subsequently, 4 µl of optically pure D-limonene or L-limonene was added and the plate was covered with a spongy silicone plate (thickness 8 mm, non-permeable film at both sides, quality 2660 shore, Maag Technik AG, Dübendorf, Switzerland), perforated with 96 holes of 1.5 mm diameter (holes positioned exactly above the centers of the wells) and a rigid stainless steel lid (Kühner AG, Basel, Switzerland). The microtiter plate and the lid were clamped together and mounted on an orbital shaker with a shaking diameter of 5 cm using equipment from Kühner AG (Basel, Switzerland). At regular time-intervals, the contents of one well was removed, centrifuged and the supernatant was analysed by LC-MS-DAD as described above. Alternatively (when indicated), cells were resuspended in 5 ml of the same buffer and transferred to a headspace vial with a total volume of 43 ml (National Scientific Company, Lawrenceville, GA). 1.0 µl of D-limonene was added as a pure compound using a microsyringe, followed by magnetic stirring at 1000 rpm and 30°C. The headspace vial was closed using a screw cap with a Teflon-coated insert. At regular time-intervals, samples of 100 µl were taken using a syringe with needle (without opening the vial in order to prevent the loss of gaseous D-limonene from the headspace), which were then centrifuged in Eppendorf tubes at 15000 rpm for 3 minutes and analyzed by LC-MS as described above.

**Biotransformation kinetics of D-limonene and L-limonene by a selection of strains grown on agar plates supplied with alkanes as the sole C-sources.** A number of selected strains were grown in Petri dishes on an agar mineral medium. After 5 days of growth in the presence of alkane vapor (as described above for the microtiter plate cultures), cell mass was scraped off the agar surface, washed and concentrated to 4 g of dry wt 1⁻¹. This cell suspension was incubated in square deepwell microtiter plates with D-limonene or L-limonene and tested for product formation after 1, 2, 4 and 6 h as described in the previous section.

### Example 1: Screening of n-alkane degraders for conversion of D-limonene and L-limonene

A total number of 137 alkane degrading strains of bacteria were tested systematically for their ability to oxidize D-limonene or L-limonene. For this purpose a miniaturised parallel growth system in microtiter plates was used. A total number of 43 strains were found to be able to oxidize the methyl group of D-limonene and/or L-limonene and gave rise to either perillyl alcohol, perillyl aldehyde, perillic acid or a mixture of two or three of these compounds (Table 1, 2 & 3). Most of these positive strains (29) were found to belong to the CNM-group (including the genera *Corynebacterium, Arthrobacter, Rhodococcus, Nocardia,* and *Mycobacterium),* as was determined by 16S DNA sequencing and/or microscopic studies after Gram staining. None of the (identified) Gram-negative strains gave rise to significant levels of any of these compounds. A number of strains were selected for further kinetic studies.

### Example 2: Biotransformation of D-limonene and L-limonene to perillyl alcohol by Rhodococcus sp. ALK2-E1 and Rhodococcus sp. HXN-1900

*Rhodococcus* sp. ALK2-E1 and *Rhodococcus* sp. HXN-1900 were selected because of their ability to convert D-limonene and L-limonene to perillyl alcohol without significant through-conversion to the aldehyde and the acid as shown in the primary screening (Table 1). Kinetic experiments on a larger scale confirmed this feature for both strains (Fig. 1 & 2): only trace amounts of perillic acid and perillyl aldehyde were formed while the concentration of perillyl alcohol was steadily increasing in the first four hours of incubation. The specific activities of perillyl alcohol formation were 0.3 and 6 U (g dry wt)⁻¹ for *Rhodococcus* sp. ALK2-E1 (from D-limonene) and *Rhodococcus* sp. HXN1900 (from L-limonene) respectively.

### Example 3. Biotransformation of D-limonene and L-limonene to perillyl alcohol and perillic acid by Mycobacterium sp. HXN-1500 and Sphingomonas sp. HXN-200

The strain studied in most detail was *Mycobacterium* sp. HXN-1500, mainly because it showed the highest specific activities of perillyl alcohol formation, and had about an equal activity on D-limonene and L-limonene (13 and 10 U (g dry wt)⁻¹ respectively, see also Fig. 3). Part of the perillyl alcohol formed was found to be through-converted to mainly perillic acid (for L-limonene also traces of perillyl aldehyde were detected). After the first 30 minutes, the detected concentrations of perillic acid were 23% and 8% of the perillyl alcohol levels for D-limonene and L-limonene respectively. Apparently, the responsible dehydrogenases had a higher activity on (+) perillyl alcohol than on (-) perillyl alcohol. The relative amounts of perillic acid gradually increased in the course of the experiment (Fig. 3). In another experiment (Fig. 4), a small amount of D-limonene (1 ul, 6.2 µmol) was incubated with 5 ml cells in a closed stirred vessel in order to determine the yield of bioconversion. At the end of the experiment, 87% of the the D-limonene added had been converted to perillic acid. In a third set of experiments (Fig. 5), a washed octane-grown cell suspension (pH 7.0) of *Mycobacterium* sp. HXN1500 (0.4 ml, 6 g dry wt 1⁻¹) was supplied with 0.1 ml pure D-limonene. In this situation, practically all perillyl alcohol formed is extracted into the limonene phase. Almost no perillic acid is extracted into the limonene phase since at pH 7.0 more than 99% of the perillic acid is deprotonated and not well soluble in the D-limonene phase.

*Sphinghomonas* sp. HXN-200 was also able to convert D-limonene and L-limonene to a mixture of perillyl alcohol and perillic acid.

### Example 4. Biotransformation of D-limonene to perillic acid by Rhodococcus sp. ALK2-C7

One strain that yielded practically solely perillic acid in the primary screening *(Rhodococcus* sp. ALK2-C7) was selected for a more detailed study as well. In the kinetic experiments (Fig. 5), also only perillic acid was formed. The specific activity of perillic acid formation was 0.3 U (g dry wt)⁻¹.

### Example 5. Biotransformation kinetics of structural analogues of limonene to the corresponding alcohols by Mycobacterium sp. HXN-1500

The following compounds with an identical carbon skeleton as limonene were also incubated with induced cells of *Mycobacterium sp.* HXN-1500 and were found to give rise to compounds with LC-mass spectra and retention times corresponding to compounds in which the methyl group (C7) has been hydroxylated in the same way as described for D-limonene and L-limonene:
(lR)-(+)-trans-isolimonene (CAS 5113-87-1);
alpha-terpinene (CAS 99-86-5);
gamma-terpinene (CAS 99-85-4);
alpha-phellandrene, racemic mixture (CAS 99-83-2);
R(-) alpha- phellandrene (CAS 4221-98-1);
terpinolene (CAS 586-62-9);
(+)-*p*-menth-1-ene(CAS 1195-31-9);
(+)- trans-*p*-menth-2-ene (CAS 5113-93-9);
4-methyl-1-iso-propylcyclohexene (CAS 500-00-5);
*p*-menthane (CAS 99-82-1);
dehydro p-cymene (CAS 1195-32-0);
2-carene (CAS 554-61-0);
(+)-2-Carene (CAS 4497-92-1);
delta-3-carene (CAS 13466-78-9); and
(+)-3-Carene (498-15-7).

The formed compounds may have pharmaceutical relevance as they are structural analogues of the anti-cancer drug perillyl alcohol.

**Table 1.**

| Strains converting D-limonene and L-limonene predominantly to perillyl alcohol (perillic acid and/or perillyl aldehyde produced at less than 20% of the concentration of perillyl alcohol) | | | | | |
|---|---|---|---|---|---|
| taxonomy | strain code | substr. | [perillyl alcohol] (µM) | [perillyl aldehyde] (µM) | [perillic acid] (µM) |
| CNM | ALK2-E1 | D-lim | 56 | b.d.^{a)} | b.d. |
| | | L-lim | 42 | b.d. | b.d. |
| CNM | ALK2-E3 | D-lim | 57 | b.d. | b.d. |
| | | L-lim | 34 | b.d. | b.d. |
| - | ALK1-1D | D-lim | 63 | b.d. | b.d. |
| | | L-lim | 47 | b.d. | b.d. |
| *Rhodococcus fascians* | ALK1-115H | D-lim | 54 | b.d. | b.d. |
| | | L-lim | 32 | b.d. | b.d. |
| *R. erythropolis* | ALK1-350 | D-lim | 151 | b.d. | 12 |
| | | L-lim | 75 | b.d. | 26 |
| *Rhodococcus fascians* | ALK1-154S | D-lim | 109 | b.d. | b.d |
| | | L-lim | 108 | b.d. | b.d. |
| *Mycobacterium* sp. | HXN-500 | D-lim | 103 | b.d. | b.d. |
| | | L-lim | 10 | b.d. | b.d. |
| *Mycobacterium* sp. | HXN-600 | D-lim | 66 | b.d. | 10 |
| | | L-lim | 51 | b.d. | b.d. |
| CNM | HXN-1000 | D-lim | 289 | b.d. | b.d. |
| | | L-lim | 100 | b.d. | b.d. |
| *Rhodococcus* sp. | HXN-1900 | D-lim | 232 | b.d. | b.d. |
| | | L-lim | 210 | b.d. | b.d. |

| | | | | | |
|---|---|---|---|---|---|
| a) b.d. = below detection limit (∼5 µM) | | | | | |

**Table 2.**

| Strains converting D-limonene and L-limonene to a mixture of perillyl alcohol, perillic acid and perillyl aldehyde (none of the compounds formed at a concentration of more than 80% of the summed concentrations of the three compounds) | | | | | |
|---|---|---|---|---|---|
| taxonomy | strain acid] | substr. code | [perillyl (µM) | [perillyl alcohol] (µM) | [perillic aldehyde] (µM) |
| CNM | ALK2-A8 | D-lim | 5 | b.d. | 37 |
| | | L-lim | 102 | b.d. | 10 |
| CNM | ALK2-A12 | D-lim. | 48 | b.d | 427 |
| | | L-lim. | 119 | - | 158 |
| CNM | ALK2-B3 | D-lim. | 113 | b.d. | 42 |
| | | L-lim. | 46 | b.d. | 46 |
| CNM | ALK2-B7 | D-lim | 38 | b.d. | 29 |
| | | L-lim | 12 | b.d. | 56 |
| CNM | ALK2-B9 | D-lim | 138 | b.d. | 406 |
| | | L-lim | 22 | b.d. | 347 |
| - | ALK2-C11 | D-lim | 14 | b.d. | 79 |
| | | L-lim | 23 | b.d. | 55 |
| *R. erythropolis* | ALK1-23D | D-lim | 79 | 27 | 32 |
| | | L-lim | 19 | 20 | 36 |
| - | ALK1-114H | D-lim | 14 | b.d. | 52 |
| | | L-lim | 13 | b.d. | 18 |
| - | ALK1-36O | D-lim | 81 | 10 | 21 |
| | | L-lim | 14 | 11 | 14 |
| *R. erythropolis* | ALK1-42O | D-lim | 23 | 12 | 71 |
| | | L-lim | 13 | 15 | 59 |
| - | ALK1-43O | D-lim | 12 | b.d. | b.d. |
| | | L-lim | 45 | 19 | 117 |
| - | ALK1-18V | D-lim | b.d. | b.d. | 115 |
| | | L-lim | 18 | b.d. | 54 |
| - | ALK1-33V | D-lim | 20 | b.d. | 67 |
| | | L-lim | 24 | b.d. | 20 |
| - | ALK1-PN3 | D-lim | b.d. | b.d. | 234 |
| | | L-lim | 42 | b.d. | 118 |
| *R. erythropolis* | NRLL B-16531 | D-lim | 98 | 14 | 25 |
| | | L-lim | 30 | 16 | 45 |
| *Mycobacterium* sp. | HXN-1500 | D-lim | 174 | b.d. | 175 |
| | | L-lim | 178 | b.d. | 54 |

**Table 3.**

| Strains converting D-limonene and L-limonene predominantly to perillic acid (perillyl alcohol and/or perillyl aldehyde produced at less than 20% of the concentration of perillyl acid) | | | | | |
|---|---|---|---|---|---|
| taxonomy | strain code | substr. | [perillyl alcohol] (µM) | [perillyl aldehyde] (µM) | [perillic acid] (µM) |
| | ALK2-A4 | D-lim | b.d. | b.d. | 56 |
| | | L-lim | b.d. | b.d. | 63 |
| CNM | ALK2-B1 | D-lim | b.d. | b.d. | 139 |
| | | L-lim | b.d. | b.d. | 157 |
| CNM | ALK2-B6 | D-lim | b.d. | b.d. | 67 |
| | | L-lim | b.d. | b.d. | b.d. |
| CNM | ALK2-B8 | D-lim | 79 | b.d. | 680 |
| | | L-lim | 12 | b.d. | 540 |
| CNM | ALK2-B10 | D-lim | 20 | b.d. | 315 |
| | | L-lim | 14 | b.d. | 128 |
| CNM | ALK2-B12 | D-lim | b.d. | b.d. | 173 |
| | | L-lim | b.d. | b.d. | 168 |
| CNM | ALK2-C1 | D-lim | 72 | b.d. | 452 |
| | | L-lim | b.d. | b.d. | 284 |
| CNM | ALK2-C2 | D-lim | b.d. | b.d. | 713 |
| | | L-lim | 109 | b.d. | 713 |
| CNM | ALK2-C4 | D-lim | 26 | b.d. | 230 |
| | | L-lim | 13 | b.d. | 109 |
| CNM | ALK2-C5 | D-lim | 22 | b.d. | 132 |
| | | L-lim | b.d. | b.d. | 55 |
| CNM | ALK2-C7 | D-lim | 9 | b.d. | 541 |
| | | L-lim | 54 | b.d. | 397 |
| - | ALK2-C8 | D-lim | 4 | b.d. | 69 |
| | | L-lim | b.d. | b.d. | 56 |
| - | ALK2-C10 | D-lim | 20 | b.d. | 170 |
| | | L-lim | 14 | b.d. | 86 |
| CNM | ALK2-F12 | D-lim | b.d. | b.d. | 56 |
| | | L-lim | b.d. | b.d. | 40. |
| CNM | ALK2-G1 | D-lim | b.d. | b.d. | 103 |
| | | L-lim | b.d. | b.d. | 63 |
| - | ALK1-66O | D-lim | b.d. | b.d. | 39 |
| | | L-lim | 16 | b.d. | 90 |
| - | ALK1-PR2 | D-lim | 10 | b.d. | 197 |
| | | L-lim | b.d. | b.d. | 129 |

## Claims

1. A process for the preparation of a hydroxymethylated terpene analog, which process comprises contacting a terpene analog having a terminal methyl group with a microbial cell or a lysate thereof, which microbial cell is capable of expressing a monooxygenase capable of terminal hydroxylation of an *n*-alkane, under conditions such that hydroxylation of the terpene analog having a terminal methyl group to a hydroxymethylated terpene analog occurs.

2. A process according to claim 1, wherein the hydroxymethylated terpene analog is perillyl alcohol and the terpene analog having a terminal methyl group is D- or L-limonene.

3. A process according to claim 1 or 2, wherein the microbial cell is a fungal, bacterial or yeast cell.

4. A process according to claim 3, wherein the bacterial cell is a CNM bacterial cell of the genus *Corynebacterium, Arthrobacter, Rhodococcus, Nocardia* or *Mycobacterium.*

5. A process according to claim 4, wherein the *Rhodococcus* cell is a *Rhodococcus* sp. ALK2-E1 or *Rhodococcus* sp. HXN-1900 cell.

6. A process according to claim 5, wherein the *Mycobacterium* cell is a *Mycobacterium* sp. HXN-1500 (CBS 109282) cell.

7. A process according to claim 3, wherein the bacterial cell is from the genus *Sphingomonas*.

8. A process according to claim 7, wherein the *Sphingomonas* cell is a *Sphingomonas* sp. HXN200 cell.

9. A process according to any one of the preceding claims, wherein the microbial cell is an isolated naturally-occurring microbial cell.

10. A process according to any one of claims 1 to 9, wherein the microbial cell is a recombinant cell.

11. A process according to claim 10, wherein the recombinant cell comprises a monooxygenase-encoding polynucleotide in addition to any monooxygenase-encoding polynucleotide(s) which the naturally-occurring form of the recombinant cell may comprise and wherein the former monooxygenase polynucleotide is capable of being expressed.

12. A process according to claim 11, wherein the monooxygenase-encoding polynucleotide in addition to any monooxygenase-encoding polynucleotide(s) which the naturally-occurring form of the recombinant cell may comprise is the same as any monooxygenase-encoding polynucleotide(s) which the naturally-occurring form of the recombinant cell comprises.

13. A process according to claim 11 or 12, wherein the monooxygenase-encoding polynucleotide in addition to any monooxygenase-encoding polynucleotide(s) which the naturally-occurring form of the recombinant cell may comprise is different from any monooxygenase-encoding polynucleotide(s) which the naturally-occurring form of the recombinant cell comprises.

14. A process according to any one of the preceding claims, wherein the incubation is carried out at a temperature of from 15-40<C.

15. A process according to claim 14, wherein the incubation is carried out at a temperature of from 20<C to 37<C.

16. A process according to any one of the preceding claims, wherein (+) or (-) perillyl alcohol is substantially the only hydroxylation product formed.

17. A process according to any one of the preceding claims, in which any perillyl alcohol is fully or partially further converted to perillyl aldehyde or perillic acid.

18. A process according to any one of the preceding claims, wherein the terpene analog having a terminal methyl group is a cyclic monoterpene having a carbon skeleton similar or identical to that of limonene.

19. A process according to claim 18, wherein the cyclic monoterpene is (1R)-(+)-trans-isolimonene (CAS 5113-87-1), alpha-terpinene (CAS 99-86-5), gamma-terpinene (CAS 99-85-4), alpha-phellandrene, racemic mixture (CAS 99-83-2), R(-) alpha-phellandrene (CAS 4221-98-1), terpinolene (CAS 586-62-9), (+)-*p*-meth-1-ene (CAS 1195-31-9), (+)- trans-p-menth-2-ene (CAS 5113-93-9), 4-methyl-1-iso-propylcyclohexene (CAS 500-00-5), *p*-menthane (CAS 99-82-1), dehydro p-cymene (CAS 1195-32-0), 2-carene (CAS 554-61-0), (+)-2-Carene (CAS 4497-92-1), delta-3-carene (CAS 13466-78-9) or (+)-3-Carene (498-15-7).

20. Use of a microbial cell as defined in any one of claims 1 to 13 or a lysate thereof for use in the preparation of a hydroxymethylated terpene analog.

21. A microbial cell as defined in any one of claims 10 to 13 or a lysate thereof.
